# EUROPEAN PATENT SPECIFICATION

(11) **EP 2 173 350 B1**
(45) Date of publication and mention of the grant of the patent: **18.04.2012**
(21) Application number: 08782336.5
(22) Date of filing: 24.07.2008
(51) Int. Cl.: C07D 473/00, C07D 405/04, A61K 31/505

(54) **STEREOSELECTIVE PROCESS FOR PREPARING PURINE DIOXOLANE NUCLEOSIDE DERIVATIVES**
STEREOSELEKTIVES VERFAHREN ZUR HERSTELLUNG VON PURINDIOXOLAN-NUKLEOSIDE-DERIVATEN
PROCÉDÉ STÉRÉOSÉLECTIF POUR PRÉPARER DES DÉRIVÉS DE PURINE DIOXOLANE NUCLÉOSIDE

(30) Priority: 30.07.2007 US 962550 P
(43) Date of publication of application: 14.04.2010
(73) Proprietor: RFS Pharma, LLC., Tucker GA 30084 (US)
(72) Inventor: COATS, Steven, J., Mcdonough, GA 30252 (US)
(74) Representative: Harrison Goddard Foote
(86) International application number: PCT/US2008/071069
(87) International publication number: WO 2009/018106

(56) References cited:
- WO-A1-97/21706
- US-A- 5 276 151
- US-A1- 2003 060 476
- US-B2- 6 743 910

## Description

### Field Of The Invention

The present invention relates to a stereoselective process for producing purine dioxolane nucleoside intermediates and purine dioxolane nucleoside analogues.

### Background Of The Invention

Nucleoside analogs as a class have a well-established regulatory history, with more than 10 currently approved by the US Food and Drug Administration (US FDA) for treating human immunodeficiency virus (HIV), hepatitis B virus (HBV), or hepatitis C virus (HCV). Significant biological activity has been demonstrated in dioxolane nucleoside analogues in which a substituted 1,3-dioxolane has replaced the carbohydrate found in natural nucleosides.

The first dioxolane analogues were reported by Belleau et al. in EP 0337713 published Oct. 18, 1989. Gu et al. reported [(2R/S,4R/S)-[4-(2,6-diamino-9*H*-purin-9-yl)-1,3-dioxolan-2-yl]methanol [(-/+)-DAPD] and 2-amino-9-((2R/S,4R/S)-2-(hydroxymethyl)-1,3-dioxolan-4-yl)-9*H*-purin-6-ol [(-/+)-DXG] to have useful efficacy against HIV-1 in various cell system (Antimicrob. Agents Chemother. 1999, 43, 2376-2382 and Nucleosides Nitcleotides 1999, 18, 891-2).

U.S. Patent No. 5,179,104 filed in 1990 by Chu and Shinazi, disclosed a method to obtain enantiomerically pure ß-D-1,3-dioxolane nucleosides via a stereospecific synthesis. A paper entitled "1,3-Dioxolanylpurine Nucleosides (2R,4R) and (2R,4S) with Selective Anti-HIV-1 Activity in Human Lymphocytes" was subsequently published (Kim et al. J. Med. Chem. 1993, 36, 30-7; and related J. Med. Chem. 1992, 35, 1987-95). The thirteen-step synthesis of (-)-DAPD was described from 1,6-anhydro-D-mannose in modest yield. This asymmetric synthesis includes several steps and involves difficult oxidation and purification steps.

PCT WO9721706 discloses a method for producing 1,3-dioxolane ring containing ß-nucleoside analogs. In the process, high stereoselectivities (α/ß ratio) are only observed when the coupling reaction is carried out at low temperatures (-78 °C). This disadvantage along with the poor yields and very long reaction times (>24 h) when 2,6-diaminopurine is the base make this process unsuitable for commercial development.

Glycosylation of 2,6-diaminopurines or silyl 2,6-diaminopurines with dioxolane acetates is reported to be facilitated by the presence of an optionally silylated 1,3-dicarbonyl compound during glycosylation reaction (US2006/0211855). This method suffers in that only a 33% yield of the desired cis isomer is obtained during the critical glycosylation step limiting its usefulness for commercial development.

It would be advantageous to have a cost-effective process scale method for preparing purine dioxolane nucleoside derivatives in racemic or optically pure form. The present invention provides such a method.

### Summary of the Invention

A cost-effective process scale method for preparing purine dioxolane nucleoside derivatives in racemic or optically pure form is disclosed, as are nucleoside derivatives prepared by the method. The method involves reacting a purine or a mono- or polysilylated purine derivative with an activated dioxolane analog to produce the dioxolane nucleoside analog in commercially useful yields.

The invention is based on the surprising discovery that direct reaction of purine or a mono- or polysilylated purine with dioxolanes takes place with highest reported chemical yields and excellent stereoselectivity when an additive in the form of an alpha cyano carbonyl compound or silylated alpha cyano carbonyl compounds is present in the reaction mixture during the glycosylation reaction.

The present invention relates to a process for preparing dioxolane compounds of the general formula (1) 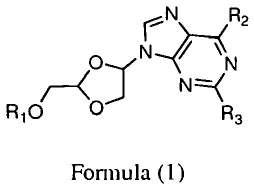 and pharmaceutically acceptable salts or prodrug thereof; wherein, R₁ is a hydroxyl protecting group; R₂ and R₃ are chosen independently from H, halogen, CN, N₃, NO₂, OH, NH₂, SH, OR', NHR', N(R')₂, SR', OCOR', NHCOR', N(COR')COR', SCOR', OCOOR', NHCOR', CH₂OH, CH₂CN, CH₂N₃, COOH, COOR', CONH₂, CONHR, CON(R')₂, CH₂COOH, CH₂COOR', CH₂CONH₂, CH₂CONHR', CH₂CON(R')₂, C₁₋₆ alkyl, C₂₋₆ alkenyl, and C₂₋₆ alkynyl, C₃₋₈ cycloalkyl, aryl, heteroaryl, acyl, arylalkyl, and alkylaryl;
each R' is independently a lower alkyl of C₁-C₆, lower cycloalkyl of C₁-C₆, aryl, alkylaryl, or arylalkyl
by reacting a compound of the general formula (2) 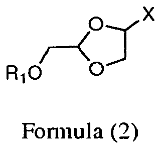 wherein X is a leaving group as defined according to J. March, "Advanced Organic Chemistry", 3rd edition, Wiley 1985
with a 2,6-substituted purine derivative of the general formula (5) 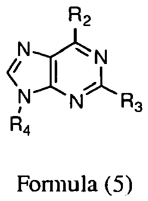 wherein; R₄ is a silyl radical,
in the presence of a Lewis acid, solvent, and additionally in the presence of a 2-cyanoethanoate compound or a silylated derivative of a 2-cyanoethanoate compound.

The process of the invention can be used to produce racemic compounds of general formula (1) and optically pure or enriched compounds through choice of precursors having an appropriate optical configuration.

The hydroxyl protecting group R₁ can be selected from all alcohol protecting group known and suitable to one skilled in the art. For example, alcohols protecting groups as described in "T. W. Greene, P. G. M. Wuts, "Protective Groups in Organic Synthesis", 3rd edition, Wiley 1999, pp. 17-200.

Leaving groups X are preferably selected from the group comprising iodine, bromine, C₁₋₂₀ acyloxy radical, C₁₋₂₀ alkylsulfonyloxy radical, C₁₋₂₀ arylsulfonyloxy radical, C₁₋₂₀alkoxyradical or C₁₋₂₀ aryloxy radical.

The 2,6-disubstituted purine derivative of the general formula (5) contains at least one C₁₋₂₀ silyl radical R₄, and optionally further silyl radicals on functions in positions 2 and 6, when possible, to act as amino protective groups.

The alpha cyano carbonyl compound used is a 2-cyanoethanoate ester, a 2-cyano ketone or a 2-cyanoethanoic acid derivative having 5 to 20 C atoms of the general formula (3) 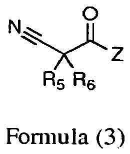 wherein Z may be hydrogen, an alkyl radical having from 1 to 20 C atoms, an aryl radical having from 6 to 20 C atoms or an alkyloxy group having from 1 to 20 C atoms and R₅ and R₆ may be independently a hydrogen, an acyl radical of an aromatic or aliphatic carboxylic acid having from 2 to 20 C atoms, an alkyl radical having from 1 to 20 C atoms or an aryl radical having from 6 to 20 C atoms.

The silylated derivative of 2-cyanoethanoate ester compound used is a silyl derivative of a 2-cyanoethanoate ester, of a 2-cyano ketone or of a 2-cyanoethanoic acid derivative of the general formula (4) 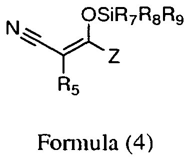 wherein Z and R₅ have the meaning set forth in claim 6, and R₇, R₈ and R₉ may be independently of one another an aliphatic or aromatic radical having from 1 to 20 C atoms.

In general all aprotic organic solvents can be used for the process. The reaction is preferably carried out under atmospheric pressure at a temperature between -25 °C and the boiling point of the solvent.

The present invention also provides a recrystallization process for purifying compounds of the general formula (I) obtained by the process of the invention.

Preferred methods for removing OH protective acyl radical groups are reaction with ammonia, aliphatic amines, basic aqueous hydrolysis, or reaction with alcoholates.

### Detailed Description of the Invention

The present invention relates to a process for preparing dioxolane compounds of the general formula (1) 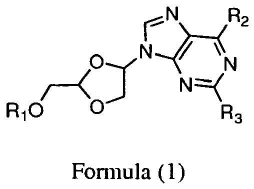 and pharmaceutically acceptable salts or prodrug thereof; wherein, R₁ is a hydroxyl protecting group; R₂ and R₃ is chosen independently from H, halogen, CN, N₃, N0₂, OH, NH₂, SH, OR', NHR', N(R')₂, SR', OCOR', NHCOR', N(COR')COR', SCOR', OCOOR', NHCOR', CH₂OH, CH₂CN, CH₂N₃, COOH, COOR', CONH₂, CONHR, CON(R')₂, CH₂COOH, CH₂COOR', CH₂CONH₂, CH₂CONHR', CH₂CON(R')₂, C₁₋₆ alkyl, C₂₋₆ alkenyl, and C₂₋₆ alkynyl, C₃₋₈ cycloalkyl, aryl, heteroaryl, acyl, arylalkyl, and alkylaryl;
each R' is independently a lower alkyl of C₁-C₆, lower cycloalkyl of C₁-C₆, aryl, alkylaryl, or arylalkyl
by reacting a compound of the general formula (2) 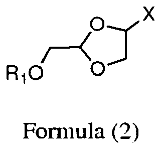 wherein X is a leaving group as defined according to J. March, "Advanced Organic Chemistry", 3rd edition, Wiley 1985
with a 2,6-substituted purine derivative of the general formula (5) 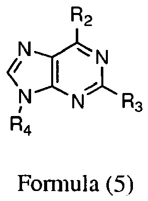 wherein; R₄ is a silyl radical,
in the presence of a Lewis acid, solvent, and additionally in the presence of a 2-cyanoethanoate compound or a silylated derivative of a 2-cyanoethanoate compound.

The process of the invention can be used to produce racemic compounds of general formula (1) and optically pure or enriched compounds obtained in the optical configuration of the general formulas (1a), (1b), (1c), or (1d)

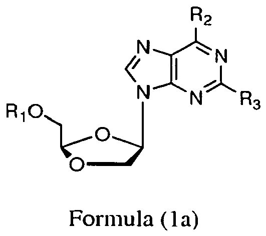

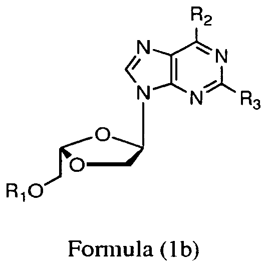

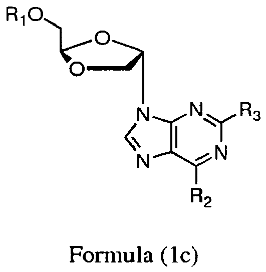

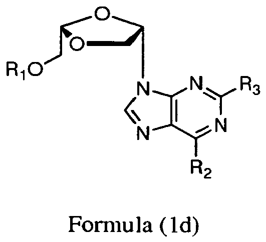

High stereoselectivity can be obtained by the process through choice of precursors having an appropriate optical configuration.

The hydroxyl protecting group R₁ can be selected from all alcohol protecting group known and suitable to one skilled in the art. For example, alcohols protecting groups as described in "T. W. Greene, P. G. M. Wuts, "Protective Groups in Organic Synthesis", 3rd edition, Wiley 1999, pp. 17-200. The hydroxyl protective groups R₁ are preferably selected from the group comprising C₂₋₂₀ acyl radicals, C₁₋₂₀ alkyl radicals, C₁₋₂₀ alkoxyalkyl radicals, C₁₋₂₀ arylalkylradicals, C₁₋₂₀ arylalkoxyalkyl radicals or C₁₋₂₀ silyl radicals.

Leaving groups X are preferably selected from the group comprising iodine, bromine, C₁₋₂₀ acyloxy radical, C₁₋₂₀ alkylsulfonyloxy radical, C₁₋₂₀ arylsulfonyloxy radical, C₁₋₂₀ alkoxyradical or C₁₋₂₀ aryloxy radical. Particular preference is given for iodine and radicals from the group comprising acetoxy-, benzoyloxy-, propionyloxy-, n-butyryloxy- and trifluoroacetoxy-. Acetoxy- is very particularly preferred.

The 2,6-disubstituted purine derivative of the general formula (5) contains at least one C₁₋₂₀ silyl radical R₄, and optionally further silyl radicals on functions in positions 2 and 6, when possible, to act as amino protective groups. A persilylated precursor of the general formula (5) may in this connection comprise up to 5 identical or different silyl radicals. For example, 2,6-diaminopurine derivatives of the general formula (5) having one to three silyl radicals are preferred, and those having three silyl radicals are very particularly preferred, especially having silyl radical on the nitrogen in position 9 and a silyl radical on each of the two amino functions in positions 2 and 6. Trimethylsilyl- is particularly preferred.

Prefered Lewis acid compounds are selected from the group comprising trialkylsilylhalides or trialkylsilyl perfluoroalkanesulfonates. Iodotrimethylsilane and trimethylsilyl trifluoromethanesulfonate are particularly preferred.

The alpha cyano carbonyl compound used is a 2-cyanoethanoate ester, a 2-cyano ketone or a 2-cyanoethanoic acid derivative having 5 to 20 C atoms of the general formula (3) 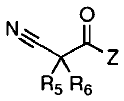 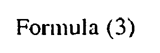 wherein Z may be hydrogen, an alkyl radical having from 1 to 20 C atoms, an aryl radical having from 6 to 20 C atoms or an alkyloxy group having from 1 to 20 C atoms and R₅ and R₆ may be independently of one another hydrogen, an acyl radical of an aromatic or aliphatic carboxylic acid having from 2 to 20 C atoms, an alkyl radical having from 1 to 20 C atoms or an aryl radical having from 6 to 20 C atoms.

The silylated derivative of 2-cyanoethanoate ester compound used is a silyl derivative of a 2-cyanoethanoate ester, of a 2-cyano ketone or of a 2-cyanoethanoic acid derivative of the general formula (4) 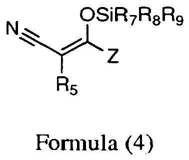 wherein Z and R₅ have the meaning set forth in claim 6, and R₇, R₈ and R₉ may be independently of one another an aliphatic or aromatic radical having from 1 to 20 C atoms.

In general all aprotic organic solvents can be used. Examples of suitable solvents are methylene chloride, 1,2-dichloroethane, and acetonitrile. Particularly preferred are methylene chloride and 1,2-dichloroethane.

The reaction is preferably carried out under atmospheric pressure at a temperature between -25 °C and the boiling point of the solvent. A temperature between -10 °C and +30 °C is preferably used.

The present invention also provides a recrystallization process for purifying compounds of the general formula (I) obtained by the process of the invention. Alcohols, ethers, or esters having 1-10 carbon atoms or other polar solvents are particularly suitable for the recrystallization. Isopropanol is particularly preferred as solvent for the recrystallization of compounds of the general formula (1) where R₁=(CH₃)₂CHCO-.

Preferred methods for removing OH protective acyl radical groups are reaction with ammonia, aliphatic amines, basic aqueous hydrolysis, or reaction with alcoholates such as, for example, sodium methoxide.

### Examples

The present invention is further illustrated in the following example. Scheme 1 shows the preparative method for synthesizing purine dioxolane nucleoside derivatives. It will be understood by one of ordinary skill in the art that these examples are in no way limiting and that variations of detail can be made without departing from the spirit and scope of the present invention.

The terms used in describing the invention are commonly used and known to those skilled in the art. As used herein, the following abbreviations have the indicated meanings:

| | |
|---|---|
| Ac | acetyl |
| DMAP | 4-dimethylaminopyridine |
| DMSO | dimethylsulfoxide |
| h | hour/hours |
| M | molar |
| min | minute |
| rt | room temperature |
| TBDMSCl | tert-butyl dimethyl silyl chloride |
| THF | tetrahydrofuran |
| TMSI | trimethylsilyl iodide |

Specific compounds which are representative of this invention were prepared as per the following examples and reaction sequences; the examples and the diagrams depicting the reaction sequences are offered by way of illustration, to aid in the understanding of the invention and should not be construed to limit in any way the invention set forth in the claims which follow thereafter. The present compounds can also be used as intermediates in subsequent examples to produce additional compounds of the present invention. No attempt has necessarily been made to optimize the yields obtained in any of the reactions. One skilled in the art would know how to increase such yields through routine variations in reaction times, temperatures, solvents and/or reagents.

Anhydrous solvents were purchased from Aldrich Chemical Company, Inc. (Milwaukee). Reagents were purchased from commercial sources. Unless noted otherwise, the materials used in the examples were obtained from readily available commercial suppliers or synthesized by standard methods known to one skilled in the art of chemical synthesis. Melting points (mp) were determined on an Electrothermal digit melting point apparatus and are uncorrected. ¹H and ¹³C NMR spectra were taken on a Varian Unity Plus 400 spectrometer at room temperature and reported in ppm downfield from internal tetramethylsilane. Deuterium exchange, decoupling experiments or 2D-COSY were performed to confirm proton assignments. Signal multiplicities are represented by s (singlet), d (doublet), dd (doublet of doublets), t (triplet), q (quadruplet), br (broad), bs (broad singlet), m (multiplet). All J-values are in Hz. Mass spectra were determined on a Micromass Platform LC spectrometer using electrospray techniques. Elemental analyses were performed by Atlantic Microlab Inc. (Norcross, GA). Analytic TLC was performed on Whatman LK6F silica gel plates, and preparative TLC on Whatman PK5F silica gel plates. Column chromatography was carried out on Silica Gel or via reverse-phase high performance liquid chromatography.

### Example 1

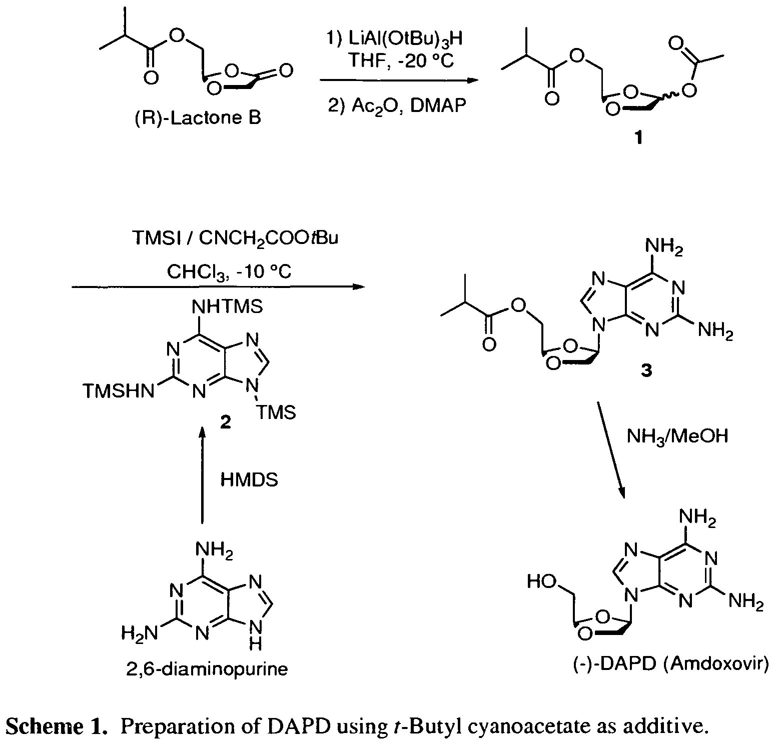

### Step 1: Silylation of 2,6-diaminopurine

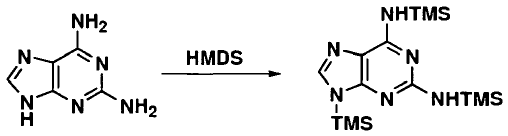

750 mg of 2,6-diaminopurine, 750 mg of ammonium sulfate and 20 mL of hexamethyldisilazane were added into a 250 mL three-neck flask. The suspension was heated to reflux with stirring at 130-135 °C (oil-bath) for 4 h. During this period the solution becomes homogeneous. The solution was cooled to 85 °C and the excess hexamethyldisilazane was subsequently distilled off under gradually decreasing reduced pressure. After the hexamethyldisilazane was removed completely, the residue was cooled to rt under vacuum then 10 mL of anhydrous methylene chloride was added to prepare a solution.

### Step 2: Preparation of (2R-4R/S)-4-acetoxy-2-isobutyryloxymethyl-1,3-dioxolane

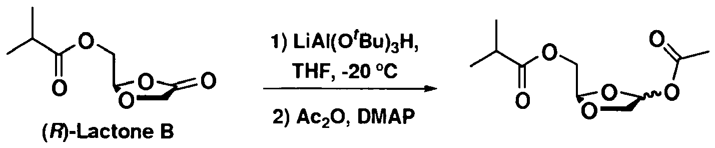

To a well stirred solution of LiAl(OtBu)₃H (25.4 g, 100 mmol) in dry THF (150 mL) at -10 to -20 °C was added a precooled isobutyricacid-4-oxo-[1,3]-dioxolan-2-(R)-yl methyl ester (12.5 g, 66 mmol) over a period of 10 min under N₂ atmosphere. The reaction mixture was allowed to stir for 2 h at -10 to -20 °C. To this solution DMAP (7.0 g, 57.4 mmol) was added in one portion and stirred for 30 min followed by dropwise addition of Ac₂O (46 mL, 443.3 mmol). After stirring the bright yellow solution for 2 h at -10 °C, the cold bath was allowed to raise to room temperature and stirred overnight at rt. The dark brown solution was pored into saturated NH₄Cl (180 mL) solution, stirred for 30 min, filtered (to remove Li salt), concentrated *in vacuo* and extracted with ethyl acetate (3 x 60 mL). The combined organic solutions were washed with saturated NaHCO₃ (2 x 50 mL), brine, dried over Na₂SO₄, filtered, and evaporated under reduced pressure to afford a crude product (red syrup). R_{f}: 0.45 (ethylacetate:hexanes 1:4). NMR showed 1: 1 mixture of α and β isomers. ¹H-NMR (CDCl₃) δ: 1.18 and 1.19 (2s, 6H, 2 x CH₃); 2.10 (s, 3H, CH₃); 4.20-4.42 (m, 4H, 2 x CH₂); 5.32 and 5.42 (t, 1H, *J*=4.4 Hz, CH); 6.41 and 6.35 (dd, 1H, *J*=4.0 Hz, *J*=1.6 Hz, CH).

Anhydrous CHCl₃ was added to a total volume of 40 mL to prepare a 1 M solution (based on a 60% yield)

### Step 3: Preparation of cis- and trans-(2R,4R)-2-isobutyryloxymethyl-4-(2,6-diaminopurin-9-yl)-[1,3]-dioxolane

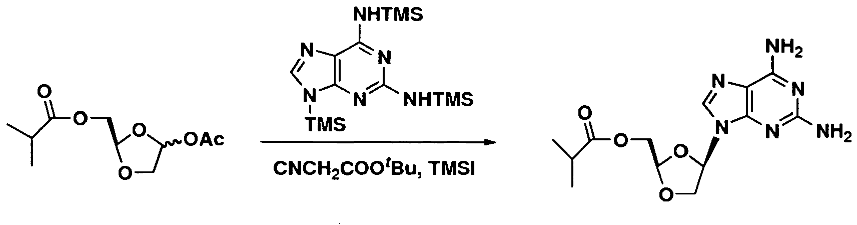

The solution of silylated 2,6-diaminopurine in dry methylene chloride (from step 1), 4 mL of 1M (2R-4R/S)-4-acetoxy-2-isobutyryloxymethyl-1,3-dioxolane solution in chloroform (from step 2) and 0.75 mL of t-butyl cyanoacetate were introduced into a dry flask. The mixture was cooled to 0 to -10 °C and, at this temperature, a solution of 1.5 mL of iodotrimethylsilane in 2 mL of methylene chloride was added dropwise over the course of 2 - 3 min. The mixture was then stirred at 0 to 5 °C for 20 h.

The reaction mixture was added dropwise to 18 mL of solution of 0.5 M hydrochloric acid at 0 °C. The mixture was warmed with stirring to 25 °C and stirred for another 20 min. The phases were separated and the organic phase was back-extracted once with 18 mL of 0.5 M hydrochloric acid. The combined aqueous phases were washed twice with 25 mL of methylene chloride. Then, after addition of a further 50 mL of methylene chloride, the pH was adjusted to 9.0 with approximately 40 mL of 10% sodium carbonate solution. The mixture was stirred at 25 °C for 1 h and the phases were separated. The aqueous phase was back-extracted twice with 30 mL of methylene chloride. The combined organic phases were washed once with 25 mL of water. Removal of the solvent in vacuum resulted in 940 mg of yellowish solid. LCMS analysis showed the isomer ratio (β:α = 2.2:1).

The crude product was recrystallized from isopropanol and 690 mg (45% yield) of colorless β-isomer crystals were obtained. NMR analysis revealed 1 mol of isopropanol in addition to (2R)-2-isobutyryloxymethyl-4-(2,6-diaminopurin-9yl)-1,3-dioxolane. ¹H-NMR (DMSO-*d*₆) δ: 0.95-1.04 (m, 12H, 4 x CH₃); 2.44-2.4 (m, 1H, CH); 3.71-3.75 (m, 1H), 4.17-4.52 (m, 5H, CH, 2 x CH₂); 5.20 (t, 1H, *J*=3.2 Hz, CH); 5.80 (brs, 2H, NH₂) 6.17 (dd, 1H, *J*=1.6 Hz, *J*=5.6 Hz, CH); 6.71 (brs, 2H, NH₂); 7.74 (s, I H, ArH).

### Step 4: Preparation of [(2R,4R)-[4-(2,6-diamino-9H-purin-9-yl)-1,3-dioxolan-2-yl]methanol [(-)-DAPD]

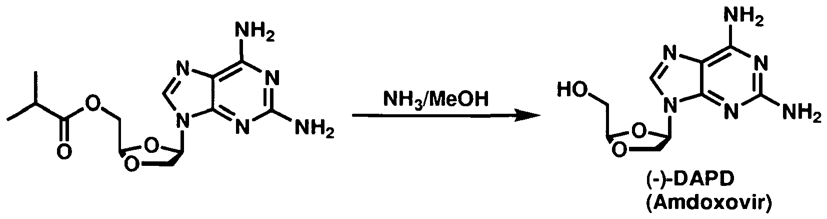

31.05 g of (2R,4R)-2-isobutyryloxymethyl-4-(2,6-diaminopurin-9-yl)-1,3-dioxolane•2-propanol was dissolved in 310 mL of NH₃-saturated methanol. The solution was stirred at 25 °C for 15 h and the solvent was distilled off in vacuo. The residue was recrystallized from ethanol/water. 17.10 g (83%) of (-)-DAPD were obtained as colorless crystals.
¹H-NMR (360 MHz, DMSO-*d*₆): d = 3.61 (dd, J₁ = 6.0 Hz, J₂ = 3.2 Hz; CH₂OH); 4.20 (dd, J₁ = 9.5 Hz, J₂ = 5.5 Hz; 1H-C(5')); 4.45 (dd, J₁ = 9.5 Hz, J₂ = 1.8 Hz; 1H-C(5')); 5.05 (Ψt, J = 3.2 Hz; 1H-C(2')); 5.15 (Ψt, J = 6.0 Hz; CH₂OH); 5.83 (s; 2H-NH₂); 6.21 (dd, J₁ = 5.5 Hz, J₂ = 1.8 Hz; 1H-C(4')); 5.83 (s; 2H-NH₂); 7.87 (s; 1H-C (8)).

Numerous references have been cited in this document. Each of these references is hereby incorporated by reference in its entirety.

This invention has been described with reference to its preferred embodiments. Variations and modifications of the invention will be obvious to those skilled in the art from the foregoing detailed description of the invention. It is intended that all of these variations and modifications be included within the scope of this invention.

## Claims

1. The invention relates to a method for the production of compounds of the general formula (1) 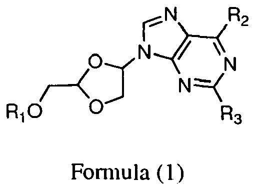 and pharmaceutically acceptable salts or prodrug thereof,
wherein R₁ is a hydroxyl protecting group;
each R₂ and R₃ is chosen independently from H, halogen, CN, N₃, NO₂, OH, NH₂, SH, OR', NHR', N(R')₂, SR', OCOR', NHCOR', N(COR')COR', SCOR', OCOOR', NHCOR', CH₂OH, CH₂CN, CH₂N₃, COOH, COOR', CONH₂, CONHR, CON(R')₂, CH₂COOH, CH₂COOR', CH₂CONH₂, CH₂CONHR', CH₂CON(R')₂, C₁₋₆ alkyl, C₂₋₆ alkenyl, and C₂₋₆ alkynyl, C₃₋₈ cycloalkyl, aryl, heteroaryl, acyl, arylalkyl, and alkylaryl; and
each R' is independently a lower alkyl of C₁-C₆, lower cycloalkyl of C₁-C₆, aryl, alkylaryl, or arylalkyl,
by reacting a compound of the general formula (2) 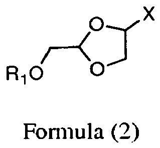 where
X is a leaving group, with a 2,6-substituted purine derivative of the general formula (5) 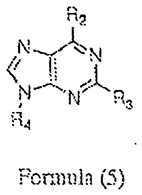 where
R₄ is silyl radical,
in the presence of a Lewis acid, solvent, and additionally in the presence of a 2-cyanoethaonate compound or a silylated derivative of a 2-cyanoethanoate compound, which forms an alpha cyano carbonyl compound in the form of a 2-cyanoethanoate ester, a 2-cyano ketone or a 2-cyanoethanoic acid derivative having 5 to 20 C atoms of the general formula (3) 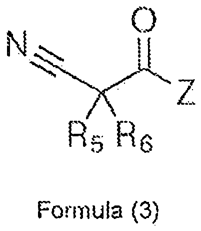 wherein Z may be hydrogen, an alkyl radical having from 1 to 20 C atoms, an aryl radical having from 6 to 20 C atoms or an alkyloxy group having from 1 to 20 C atoms and
R5 and R6 may be independently of one another hydrogen, an acyl radical of an aromatic or aliphatic carboxylic acid having from 2 to 20 C atoms, an alkyl radical having from 1 to 20 C atoms or an aryl radical having from 6 to 20 C atoms; and
the silylated derivative of 2-cyanoethanoate ester compound used is a silyl derivative of a 2-cyanoethanoate ester, of a 2-cyano ketone or of a 2-cyanoethanoic acid derivative of the general formula (4) 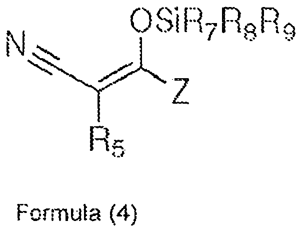 wherein Z and R5 have the meaning set forth in claim 6, and R7, R8 and R9 may be independently of one another an aliphatic or aromatic radical having from 1 to 20 C atoms.

2. The process as claimed in Claim 1, wherein the compounds of the genera! formula (1) are obtained in the optical configuration of the general formula (1a), (1b), (1c) or (1d)

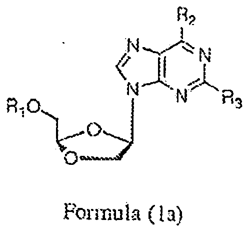

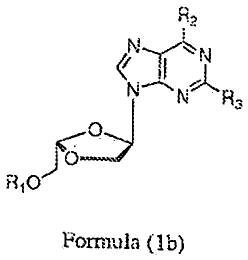

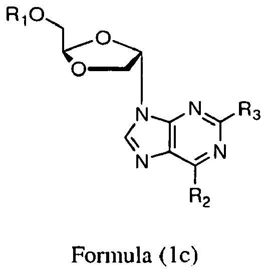

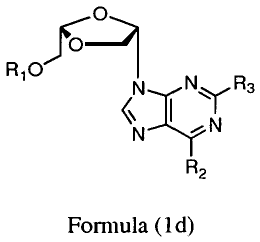

3. The process as claimed in claims 1 to 2, wherein R₁ is selected from the group comprising acyl, alkyl, alkoxyalkyl, arylalkyl, arylalkoxyalkyl or silyl.

4. The process as claimed in one or more of claims 1 to 3, wherein X is selected from the group comprising halogen, acyloxyl, alkyl-sulfonyloxyl, arylsulfonyloxyl, alkoxyl or aryloxl radicals.

5. The process as claimed in one or more of claims 1 to 4, wherein the Lewis acid comprises one or more of trialkylsilylhalides and trialkylsilyl perfluoroalkanesulfonates.

6. The process as claimed in one or more of claims 1 to 5, **characterized in that** the alpha cyano carbonyl compound used is a 2-cyanoethanoate ester, a 2-cyano ketone or a 2-cyanoethanoic acid derivative having 5 to 20 C atoms of the general formula (3) 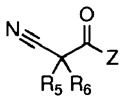 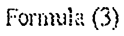 wherein
Z is hydrogen, an alkyl radical having from 1 to 20 C atoms, an aryl radical having from 6 to 20 C atoms or an alkyloxy group having from I to 20 C atoms and
R₅ and R₆ may be independently of one another hydrogen, an acyl radical of an aromatic or aliphatic carboxylic acid having from 2 to 20 C atoms, an alkyl radical having from 1 to 20 C atoms or an aryl radical having from 6 to 20 C atoms.

7. The process as claimed in one or more of claims 1 to 6, **characterized in that** the silylated derivative of 2-cyanoethanoate ester compound used is a silyl derivative of a 2-cyanoethanoate ester, of a 2-cyano ketone or of a 2-cyanoethanoic acid derivative of the general formula (4) 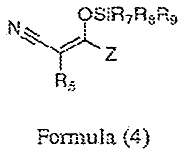 where
Z and R₅ have the meaning set forth in claim 6, and
R₇, R₈ and R₉ may be independently of one another an aliphatic or aromatic radical having from 1 to 20 C atoms.

8. The process as claimed in one or more of claims 1 to 7, wherein are or both of R₂ and R₃ are amino groups, and wherein the amino groups are protected with protective groups selected from the group comprising acyl radicals, acyloxycarbonyl radicals, alkyl radicals, arylalkyl radicals or silyl radicals.

9. The process as claimed in one or more of claims 1 to 8, wherein the resulting compounds of the general formula (1) are subsequently purified by recrystallization.

10. The process as claimed in one or more of claims 1 to 9, further comprising removing the protective group R₁ of the general formula (1) to form a compound of the general formula (6) 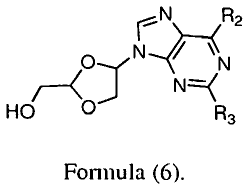 Where R₂ and R₃ have the same meaning as set forth in claim 1.

## Patentansprüche

1. Die vorliegende Erfindung betrifft ein Verfahren zur Herstellung von Verbindungen der allgemeinen Formel (1) 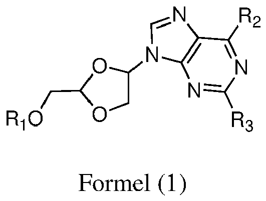 und pharmazeutisch verträglicher Salze oder Prodrugs dieser,
wobei es sich bei R₁ um eine Hydroxylschutzgruppe handelt;
wobei R₂ und R₃ unabhängig ausgewählt wird von H, Halogen, CN, N₃, NO₂, OH, NH₂, SH, OR', NHR', N(R')₂, SR', OCOR', NHCOR', N(COR')COR', SCOR', OCOOR', NHCOR', CH₂OH, CH₂CN, CH₂N₃, COOH, COOR', CONH₂, CONHR, CON(R')₂, CH₂COOH, CH₂COOR', CH₂CONH₂, CH₂CONHR', CH₂CON(R')₂, C₁₋₆ Alkyl, C₂₋₆ Alkenyl ind C₂₋₆ Alkynyl, C₃₋₈ Cycloalkyl, Aryl, Heteroaryl, Acyl, Arylalkyl, und Alkylaryl; und
wobei jedes R' unabhängig ein niedrigeres Alkyl von C₁-C₆, ein niedrigeres Cycloalkyl von C₁-C₆, Aryl, Alkylaryl oder Arylalkyl darstellt,
durch Reaktion einer Verbindung der allgemeinen Formel (2) 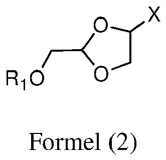 wobei
X eine Abgangsgruppe darstellt,
mit einem 2,6-substituierten Purinderivat der allgemeinen Formel (5) 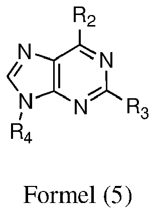 wobei
es sich bei R₄ um ein Silylradikal handelt,
in Gegenwart einer Lewis-Säure, eines Lösemittels und zusätzlich in Gegenwart einer 2-Cyanoethanoat-Verbindung oder eines silylierten Derivats einer 2-Cyanoethanoat-Verbindung, die eine Alpha-Cyanocarbonylverbindung in Form eines 2-Cyanoethanoatesters, eines 2-Cyanoketons oder eines 2-Cyanoethanolsäurederivats mit 5 bis 20 C-Atomen der allgemeinen Formel (3) bildet 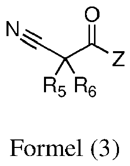 wobei es sich bei Z um Wasserstoff, ein Alkylradikal mit 1 bis 20 C-Atomen, ein Arylradikal mit 6 bis 20 C-Atomen oder eine Alkyloxy-Gruppe mit 1 bis 20 C-Atomen handeln kann; und
wobei R₅ and R₆ unabhängig voneinander Wasserstoff, ein Acylradikal einer aromatischen oder aliphatischen Carboxylsäure mit 2 bis 20 C-Atomen, ein Alkylradikal mit 1 bis 20 C-Atomen oder ein Arylradikal mit 6 bis 20 C-Atomen sein können; und
wobei es sich bei dem silylierten Derivat einer verwendeten 2-Cyanoethanoatester-Verbindung um ein Silylderivat eines 2-Cyanoethanoatesters, eines 2-Cyanoketons oder eines 2-Cyanoethanolsäurederivats der folgenden allgemeinen Formel (4) handelt 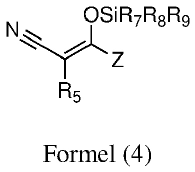 wobei Z und R₅ die in Anspruch 6 ausgeführte Bedeutung haben, und wobei R₇, R₈ und R₉ unabhängig voneinander ein aliphatisches oder aromatisches Radikal mit 1 bis 20 C-Atomen sein können.

2. Verfahren nach Anspruch 1, wobei die Verbindungen der allgemeinen Formel (1) in der optischen Konfiguration der allgemeinen Formel (1a), (1b), (1c) oder (1d) erhalten werden

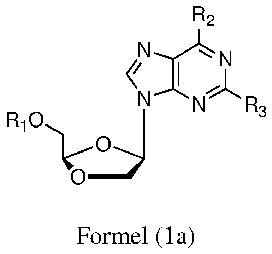

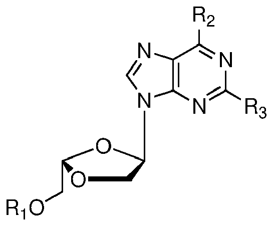

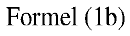

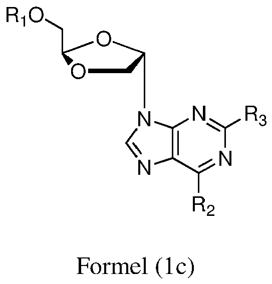

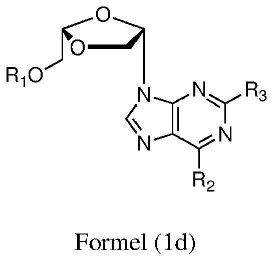

3. Verfahren nach Anspruch 1 oder 2, wobei R₁ aus der Gruppe ausgewählt wird, die Acyl, Akyl, Alkoxyalkyl, Arylalkyl, Arylalkoxyalkyl oder Silyl umfasst.

4. Verfahren nach einem oder mehreren der Ansprüche 1 bis 3, wobei X aus der Gruppe ausgewählt wird, die Halogen, Acyloxyl, Alkyl-Sulfonyloxyl, Arylsulfonyloxyl, Alkoxyl oder Aryloxl-Radikale umfasst.

5. Verfahren nach einem oder mehreren der Ansprüche 1 bis 4, wobei die Lewis-Säure einen oder mehrere der Stoffe Trialkylsilylhalide und Trialkylsilyl-Perfluoralkanesulfonate umfasst.

6. Verfahren nach einem oder mehreren der Ansprüche 1 bis 5, **dadurch gekennzeichnet, dass** es sich bei der verwendeten Alpha-Cyanocarbonylverbindung um ein 2-Cyanoethanoatester, ein 2-Cyanoketon oder ein 2-Cyanoethanolsäurederivat mit 5 bis 20 C-Atomen der folgenden allgemeinen Formen (3) handelt 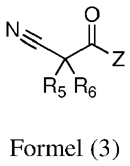 wobei
wobei es sich bei Z um Wasserstoff, ein Alkylradikal mit 1 bis 20 C-Atomen, ein Arylradikal mit 6 bis 20 C-Atomen oder eine Alkyloxy-Gruppe mit 1 bis 20 C-Atomen handeln kann; und
wobei R₅ and R₆ unabhängig voneinander Wasserstoff, ein Acylradikal einer aromatischen oder aliphatischen Carboxylsäure mit 2 bis 20 C-Atomen, ein Alkylradikal mit 1 bis 20 C-Atomen oder ein Arylradikal mit 6 bis 20 C-Atomen sein können.

7. Verfahren nach einem oder mehreren der Ansprüche 1 bis 6, **dadurch gekennzeichnet, dass** es sich bei dem verwendeten silylierten Derivat einer 2-Cyanoethanoatester-Verbindung um ein Silylderivat eines 2-Cyanoethanoatesters, eines 2-Cyanoketons oder eines 2-Cyanoethanolsäurederivats der allgemeinen Formel (4) handelt 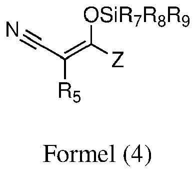 wobei
Z und R₅ die in Anspruch 6 ausgeführte Bedeutung haben, und
R₇, R₈ und R₉ unabhängig voneinander ein aliphatisches oder aromatisches Radikal mit 1 bis 20 C-Atomen sein können.

8. Verfahren nach einem oder mehreren der Ansprüche 1 bis 7, wobei R₂ und/oder R₃ Aminogruppen darstellen, und wobei die Aminogruppen durch Schutzgruppen geschützt werden, die aus der Gruppe ausgewählt werden, die Acylradikale, Acyloxycarbonyl-Radikale, Alkylradikale, Arylalkylradikale oder Silylradikale umfasst.

9. Verfahren nach einem oder mehreren der Ansprüche 1 bis 8, wobei die resultierenden Verbindungen der allgemeinen Formel (1) in der Folge durch Rekristallisation gereinigt werden.

10. Verfahren nach einem oder mehreren der Ansprüche 1 bis 9, wobei dieses das Entfernen der Schutzgruppe R₁ der allgemeinen Formel (1) umfasst, so dass eine Verbindung der allgemeinen Formel (6) gebildet wird 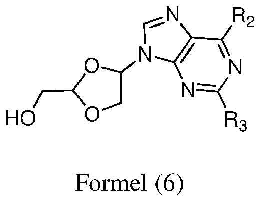 wobei R₂ und R₃ die gleiche Bedeutung wie in Anspruch 1 aufweisen.

## Revendications

1. L'invention concerne un procédé de production de composés de la formule
générale (1) 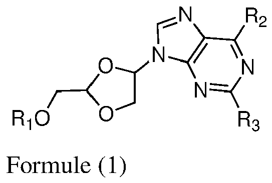 et de sels pharmaceutiquement acceptables ou d'un promédicament de ceux-ci,
dans laquelle R₁ est un groupe protecteur hydroxyle ;
chaque R₂ et R₃ est choisi indépendamment parmi H, halogène, CN, N₃, NO₂, OH, NH₂, SH, OR', NHR', N(R')_{2,} SR', OCOR', NHCOR', N(COR')COR', SCOR', OCOOR', NHCOR', CH₂OH, CH₂CN, CH₂N₃, COOH, COOR', CONH₂, CONHR, CON(R')₂, CH₂COOH, CH₂COOR', CH₂CONH₂, CH₂CONHR', CH₂CON(R')₂, alkyle en C_{1-6,} alkényle en C₂₋₆ et alkynyle en C₂₋₆, cycloalkyle en C₃₋₈, aryle, hétéroaryle, acyle, arylalkyle et alkylaryle ; et chaque R' est indépendamment un alkyle inférieur de C₁₋₆, cycloalkyle inférieur de C₁₋₆, aryle, alkylaryle ou arylalkyle,
en faisant réagir un composé de la formule générale (2) 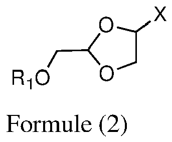 où
X est un groupe partant,
avec un dérivé de purine 2,6-substituée de la formule générale (5) 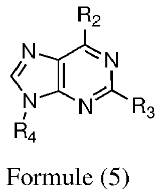 où
R₄ est un radical silyle,
en présence d'un acide de Lewis, d'un solvant et en outre en présence d'un composé 2-cyanoéthanoate ou d'un dérivé silylé d'un composé 2-cyanoéthanoate, qui forme un composé alpha cyanocarbonyle sous la forme d'un ester de 2-cyanoéthanoate, d'une 2-cyanocétone ou d'un dérivé d'acide 2-cyanoéthanoïque ayant 5 à 20 atomes de C de la formule générale (3) 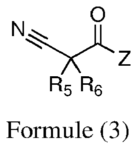 dans laquelle Z peut être hydrogène, un radical alkyle ayant 1 à 20 atomes de C, un radical aryle ayant 6 à 20 atomes de C ou un groupe alkyloxy ayant 1 à 20 atomes de C et R5 et R6 peuvent être indépendamment l'un de l'autre hydrogène, un radical acyle d'un acide carboxylique aromatique ou aliphatique ayant 2 à 20 atomes de C, un radical alkyle ayant 1 à 20 atomes de C ou un radical aryle ayant 6 à 20 atomes de C ; et
le dérivé silylé du composé ester de 2-cyanoéthanoate utilisé est un dérivé de silyle d'un ester de 2-cyanoéthanoate, d'une 2-cyanocétone ou d'un dérivé d'acide 2-cyanoéthanoïque de la formule générale (4) 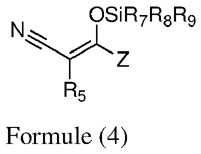 dans laquelle Z et R5 ont la signification établie à la revendication 6, et R7, R8 et R9 peuvent être indépendamment les uns des autres un radical aliphatique ou aromatique ayant 1 à 20 atomes de C.

2. Procédé selon la revendication 1, dans lequel les composés de la formule générale (1) sont obtenus dans la configuration optique de la formule générale (1a), (1b), (1c) ou (1d)

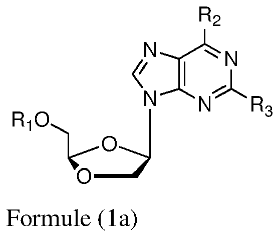

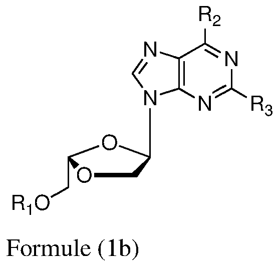

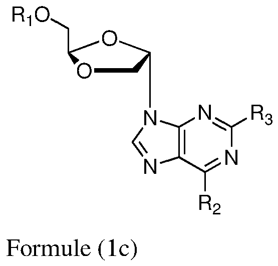

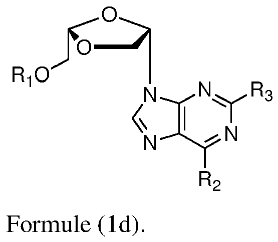

3. Procédé selon les revendications 1 à 2, dans lequel R₁ est sélectionné parmi le groupe comprenant acyle, alkyle, alcoxyalkyle, arylalkyle, arylalcoxyalkyle ou silyle.

4. Procédé selon une ou plusieurs des revendications 1 à 3, dans lequel X est sélectionné parmi le groupe comprenant des radicaux halogène, acyloxyle, alkylsulfonyloxyle, arylsulfonyloxyle, alcoxyle ou aryloxyle.

5. Procédé selon une ou plusieurs des revendications 1 à 4, dans lequel l'acide de Lewis comprend un ou plusieurs de trialkylsilylhalogénures et de perfluoroalcanesulfonates de trialkylsilyle.

6. Procédé selon une ou plusieurs des revendications 1 à 5, **caractérisé en ce que** le composé alpha cyanocarbonyle utilisé est un ester de 2-cyanoéthanoate, une 2-cyanocétone ou un dérivé d'acide 2-cyanoéthanoïque ayant 5 à 20 atomes de C de la formule générale (3) 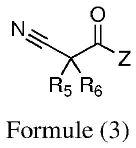 dans laquelle
Z est hydrogène, un radical alkyle ayant 1 à 20 atomes de C, un radical aryle ayant 6 à 20 atomes de C ou un groupe alkyloxy ayant 1 à 20 atomes de C et
R₅ et R₆ peuvent être indépendamment l'un de l'autre hydrogène, un radical acyle d'un acide carboxylique aromatique ou aliphatique ayant 2 à 20 atomes de C, un radical alkyle ayant 1 à 20 atomes de C ou un radical aryle ayant 6 à 20 atomes de C.

7. Procédé selon une ou plusieurs des revendications 1 à 6, **caractérisé en ce que** le dérivé silylé du composé ester de 2-cyanoéthanoate utilisé est un dérivé de silyle d'un ester de 2-cyanoéthanoate, d'une 2-cyanocétone ou d'un dérivé d'acide 2-cyanoéthanoïque de la formule générale (4) 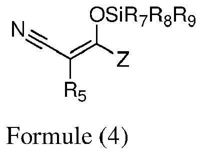 où
Z et R₅ ont la signification établie à la revendication 6, et
R₇, R₈ et R₉ peuvent être indépendamment les uns des autres un radical aliphatique ou aromatique ayant 1 à 20 atomes de C.

8. Procédé selon une ou plusieurs des revendications 1 à 7, dans lequel un de R₂ et R₃ ou les deux sont des groupes amino, et dans lequel les groupe amino sont protégés par des groupes protecteurs sélectionnés parmi le groupe comprenant des radicaux acyle, des radicaux acyloxycarbonyle, des radicaux alkyle, des radicaux arylalkyle ou des radicaux silyle.

9. Procédé selon une ou plusieurs des revendications 1 à 8, dans lequel les composés résultants de la formule générale (1) sont ensuite purifiés par recristallisation.

10. Procédé selon une ou plusieurs des revendications 1 à 9, comprenant en outre l'élimination du groupe protecteur R₁ de la formule générale (1) pour former un composé de la formule générale (6) 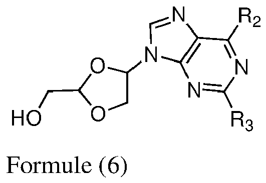 où R₂ et R₃ ont la même signification qu'établie à la revendication 1.
